# EUROPEAN PATENT APPLICATION

(11) **EP 4 282 367 A1**
(43) Date of publication of application: **29.11.2023**
(21) Application number: 21920713.1
(22) Date of filing: 18.11.2021
(51) Int. Cl.: A61B 34/37

(54) **MASTER ARM OF SURGICAL ROBOT AND SURGICAL ROBOT**

(30) Priority: 19.01.2021 CN 202110069575
(71) Applicant: Harbin Intelligent Surgery Equipment Co., Ltd., Harbin, Heilongjiang 150000 (CN)
(72) Inventor: ZHANG, Jiaxing, Harbin, Heilongjiang 150000 (CN); WANG, Jianguo, Harbin, Heilongjiang 150000 (CN)
(74) Representative: Zaboliene, Reda
(86) International application number: PCT/CN2021/131486
(87) International publication number: WO 2022/156337

(57) **Abstract**

The present invention provides a master arm of a surgical robot and the surgical robot, and relates to the technical field of medical instruments. The master arm of the surgical robot includes a first support arm, a second support arm, a third support arm, a sliding table group, a first parallelogram link mechanism and a second parallelogram link mechanism, one end of the first support arm is rotatably connected with one end of the second support arm, the other end of the second support arm is rotatably connected with one end of the third support arm, two ends of the first parallelogram link mechanism are connected with the first support arm and the third support arm respectively, and one end of the second parallelogram link mechanism is connected with the second support arm; wherein the first parallelogram link mechanism and the second parallelogram link mechanism are suitable for deforming with the swing of the second support arm, therefore, when the third support arm is connected with a surgical instrument, the swing of the surgical instrument around a telecentric point can be achieved by controlling the swing of the second support arm, and then, precise and flexible surgical operation is achieved.

## Description

### Technical Field

The present invention relates to the technical field of medical instruments, in particular to a master arm of a surgical robot and the surgical robot.

### Background Art

Most of master arms of current laparoscopic surgical robots are in a form of "fixed point", so-called "fixed point" means an opening position on the abdomen of a patient and mainly aims at avoiding the situation that the opening position on the abdomen is stressed no matter which operation is made to a focus by a surgical instrument via the opening position, and therefore, a "fixed point" structure in a laparoscopic surgical robot is very important for a surgery.

At present, a master arm is mainly implemented in the three ways described as follows: firstly, motors adopted to directly drive joints, thereby meeting the demand on the "fixed point" in the laparoscopic surgical robot; secondly, a belt or steel wire driving way is adopted; and thirdly, links are directly connected so that one point of a parallelogram is used as a moving point, and two points are transferred to a fixed point. However, there are defects in all of the above-mentioned three ways. In the first way, due to the structure itself, the state of the "fixed point" cannot be kept and is achieved by power control, in this way, uncertain motion, brought by power faults, of the master arm has to be faced, which will bring a great risk for the surgery; and the cost is high, and the complicated structure causes a high fault risk. In the second way, belt or steel wire driving is adopted, and the nature of such a driving way decides its high elasticity, low rigidity and high process implementation difficulty, thereby causing precision reduction and bringing a high surgical risk. In the third way, due to the direct connection of the links, the stress of the links in a motion range may be in sinusoidal variation, and the variation process of the stress is relatively unreasonable, thereby bringing the problems of low mechanism rigidity, power matching difficulty and electric control difficulty, which inevitably causes surgical precision reduction and surgical risk increment.

### Summary of the Invention

Problems to be solved by the present invention are poor rigidity, poor precision and electric control difficulty of a master arm in a form of a "fixed point" in a laparoscopic surgical robot.

In order to solve the above-mentioned problems, the present invention provides a master arm of a surgical robot, including a first support arm, a second support arm, a third support arm, a first parallelogram link mechanism and a second parallelogram link mechanism, one end of the first support arm being rotatably connected with one end of the second support arm, the other end of the second support arm being rotatably connected with one end of the third support arm, two ends of the first parallelogram link mechanism being connected with the first support arm and the third support arm respectively, and one end of the second parallelogram link mechanism being connected with the second support arm;
wherein the first parallelogram link mechanism and the second parallelogram link mechanism are suitable for deforming with the swing of the second support arm.

Optionally, the first parallelogram link mechanism includes two parallel first connecting joints and two parallel first links, the second parallelogram link mechanism includes two parallel second connecting joints and two parallel second links, the first connecting joints are hinged with the first links, the second connecting joints are hinged with the second links, the first connecting joint adjacent to the first support arm is fixedly connected with the first support arm, the other first connecting joint is fixedly connected with the third support arm,the second connecting joint adjacent to the second support arm is fixedly connected with the second support arm and is rotatably connected with the third support arm, and the other second connecting joint is fixedly connected with a sliding table group.

Optionally, the master arm of the surgical robot further includes a base and a multi-dimensional force sensor, the base is suitable for connection with the multi-dimensional force sensor, and the base is rotatably connected with the end, away from the second support arm, of the first support arm.

Optionally, the master arm of the surgical robot further includes harmonic reduction gears, motors and pulley sets which are disposed on two ends of the first support arm, the pulley sets include driving wheels and driven wheels which are connected in a driving way, the driving wheels are connected with the motors in a driving way, the driven wheels are connected with the harmonic reduction gears in a driving way, and two ends of the first support arm are connected with the base and the second support arm respectively via the harmonic reduction gears.

Optionally, the master arm of the surgical robot further includes a first photoelectric sensor disposed on the first support arm, the base is provided with a sensing coating or first sensing patch, and the first photoelectric sensor and the sensing coating or the first sensing patch are matched to limit a rotation angle of the first support arm relative to the base.

Optionally, the master arm of the surgical robot further includes a second photoelectric sensor and a second sensing patch, the second photoelectric sensor is disposed on the second support arm, the second sensing patch is disposed on the first support arm, and the second photoelectric sensor and the second sensing patch are matched to limit a rotation angle of the second support arm relative to the first support arm.

Optionally, the master arm of the surgical robot further includes braking mechanisms, the braking mechanisms are disposed on a junction of the base and the first support arm and a junction of the first support arm and the second support arm, the braking mechanisms include braking discs, braking rods and first electromagnets, side surfaces of the braking discs are provided with first protruding portions, side surfaces of the braking rods are provided with second protruding portions, and the first electromagnets are suitable for electrification to adsorb the braking rods so as to enable the first protruding portions and the second protruding portions to be located on the same plane and are also suitable for enabling side surfaces of the first protruding portions and the second protruding portions to be abutted.

Optionally, elastic members are disposed in the braking rods, and when the first electromagnets are electrified to adsorb the braking rods, the elastic members elastically deform so as to drive the braking rods to reset when the first electromagnets are unelectrified; or
the master arm of the surgical robot further includes second electromagnets, when the first electromagnets are unelectrified, the second electromagnets are suitable for electrification to adsorb the braking rods so that the braking rods reset.

Optionally, the master arm of the surgical robot further includes a sliding table group, the other end of the third support arm is rotatably connected with the sliding table group, the other end of the second parallelogram link mechanism is connected with the sliding table group, the sliding table group includes sliding tables, lead screws, guide rails and mounting tables, the sliding tables are rotatably connected with the third support arm and are fixedly connected with the second parallelogram link mechanism, two ends of the lead screws are rotatably connected with the sliding tables, the guide rails are fixed to the sliding tables, nuts of the lead screws are fixedly connected with the mounting tables, the mounting tables are slidably connected with the guide rails, and the mounting tables are suitable for mounting surgical instruments.

The present invention further provides a surgical robot including the above-mentioned master arm of the surgical robot.

The present invention has the beneficial effects that: two ends of the first parallelogram link mechanism are connected with the first support arm and the third support arm respectively, so that the first support arm and the third support arm swing synchronously under the limit of the first parallelogram link mechanism; one end of the second parallelogram link mechanism is connected with the second support arm, so that the second support arm and a surgical instrument swing synchronously under the limit of the second parallelogram link mechanism when the third support arm is provided with the surgical instrument; moreover, the second support arm and the third support arm form two adjacent sides of a parallelogram, an intersection point of the other two sides of the parallelogram is used as a telecentric point, namely a fixed point, and the surgical instrument mounted on the third support arm penetrates through the telecentric point; the first parallelogram link mechanism and the second parallelogram link mechanism are suitable for deforming with the swing of the second support arm, and therefore, when the second support arm swings around the first support arm, the position of the telecentric point may not vary, the surgical instrument may swing synchronously with the second support arm, and then, the surgical instrument swings around the telecentric point; however, in an actual surgical process, the telecentric point overlaps with a minimally invasive surgical incision, and then, it can be ensured that no pull occurs between the surgical instrument and a surgical incision of a patient during a minimally invasive surgery, which guarantees the safety of the surgery. Due to the layout and driving forms of the first parallelogram link mechanism and the second parallelogram link mechanism, the form variation of the two groups of parallelograms within a motion range of the structure is about 90° where the stress is optimal, so that the structural stress is optimal, and the rigidity is optimal. Moreover, links are compact in structure, high in rigidity and light in weight, so that the swing of the surgical instrument around the telecentric point can be precisely achieved by controlling the swing of the second support arm, and then, a precise and flexible surgical operation is achieved.

### Brief Description of the Drawings

Fig. 1 is an exploded view of a master arm of a surgical robot in an embodiment of the present invention;
Fig. 2 is a sectional view of the master arm of the surgical robot in the embodiment of the present invention;
Fig. 3 is an exploded view of a sliding table group of the surgical robot in the embodiment of the present invention;
Fig. 4 is an enlarged view of a part I in Fig. 2; and
Fig. 5 is an enlarged view of a part II in Fig. 2.

Descriptions for reference numerals in the accompanying drawings:
1-first support arm, 2-second support arm, 3-third support arm, 4-sliding table group, 401-sliding table, 4011-upper sliding table cover, 4012-sliding table body, 4013-lower sliding table cover, 402-lead screw, 403-guide rail, 404-mounting table, 5-first parallelogram link mechanism, 501-first connecting joint, 502-first link, 6-second parallelogram link mechanism, 601-second connecting joint, 602-second link, 7-base, 8-multi-dimensional force sensor, 9-harmonic reduction gear, 10-motor, 11-pulley set, 12-first photoelectric sensor, 13-second photoelectric sensor, 14-second sensing patch, 15-braking disc, 1501-first protruding portion, 16-braking rod, 1601-second protruding portion, 17-first electromagnet, 18-motor control panel, and 19-telecentric point.

### Detailed Description of the Invention

In order to make the above-mentioned objectives, features and advantages of the present invention more obvious and understandable, specific embodiments of the present invention will be described in detail below with reference to the accompanying drawings.

In the description of the present invention, it needs to be understood that directional or positional relationships indicated by terms such as "upper", "lower", "front" and "rear" are directional or positional relationships based on the accompanying drawings, are merely intended to facilitate describing the present invention and simplifying the description, rather than to indicate or imply that the appointed device or element has to be located in a specific direction or structured and operated in the specific direction so as not to be understood as restrictions on the present invention.

Terms such as "first" and "second" are for descriptive purposes only, and cannot be understood as indicating or implying the relative importance or implicitly indicating the number of indicated technical features. Therefore, the features defined as "first" and "second" may explicitly or implicitly include at least one of the features.

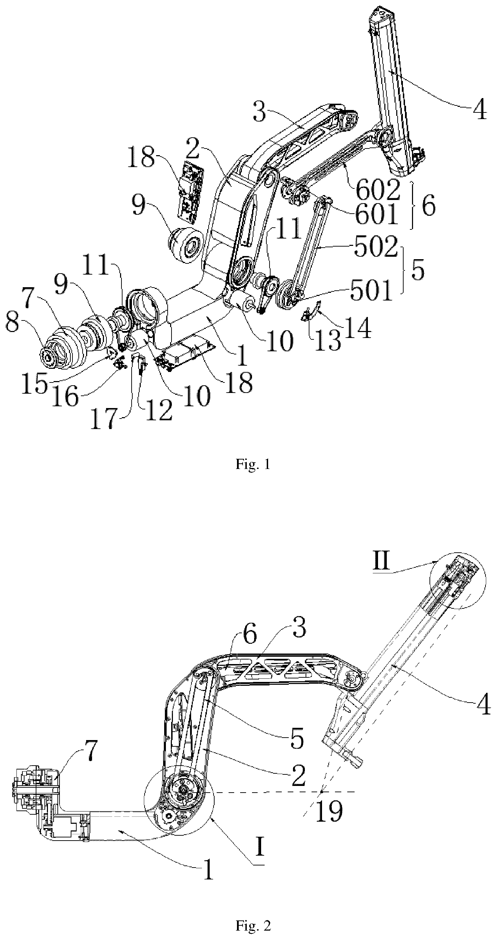

As shown in Fig. 1 and Fig. 2, a master arm of a surgical robot in an embodiment of the present invention includes a first support arm 1, a second support arm 2, a third support arm 3, a first parallelogram link mechanism 5 and a second parallelogram link mechanism 6, one end of the first support arm 1 is rotatably connected with one end of the second support arm 2, the other end of the second support arm 2 is rotatably connected with one end of the third support arm 3, two ends of the first parallelogram link mechanism 5 are connected with the first support arm 1 and the third support arm 3 respectively, and one end of the second parallelogram link mechanism 6 is connected with the second support arm 2;
wherein the first parallelogram link mechanism 5 and the second parallelogram link mechanism 6 are suitable for deforming with the swing of the second support arm 2.

Two ends of the first parallelogram link mechanism 5 are connected with the first support arm 1 and the third support arm 3 respectively, so that the first support arm 1 and the third support arm 3 swing synchronously under the limit of the first parallelogram link mechanism 5; one end of the second parallelogram link mechanism 6 is connected with the second support arm 2, so that the second support arm 2 and a surgical instrument swing synchronously under the limit of the second parallelogram link mechanism 6 when the third support arm 3 is provided with the surgical instrument; moreover, the second support arm 2 and the third support arm 3 form two adjacent sides of a parallelogram, an intersection point of the other two sides of the parallelogram is used as a telecentric point 19, namely a fixed point, and the surgical instrument mounted on the third support arm 3 penetrates through the telecentric point 19; the first parallelogram link mechanism 5 and the second parallelogram link mechanism 6 are suitable for deforming with the swing of the second support arm 2, and therefore, when the second support arm 2 swings around the first support arm 1, the position of the telecentric point 19 may not vary, the surgical instrument may swing synchronously with the second support arm 2, and then, the surgical instrument swings around the telecentric point 19; however, in an actual surgical process, the telecentric point 19 overlaps with a minimally invasive surgical incision, and then, it can be ensured that no pull occurs between the surgical instrument and a surgical incision of a patient during a minimally invasive surgery, which guarantees the safety of the surgery. Due to the layout and driving forms of the first parallelogram link mechanism 5 and the second parallelogram link mechanism 6, the form variation of the two groups of parallelograms within a motion range of the structure is about 90° where the stress is optimal, so that the structural stress is optimal, and the rigidity is optimal. Moreover, links are compact in structure, high in rigidity and light in weight, so that the swing of the surgical instrument around the telecentric point 19 can be precisely achieved by controlling the swing of the second support arm 2, and then, a precise and flexible surgical operation is achieved.

Optionally, the first parallelogram link mechanism 5 includes two parallel first connecting joints 501 and two parallel first links 502, the second parallelogram link mechanism 6 includes two parallel second connecting joints 601 and two parallel second links 602, the first connecting joints 501 are hinged with the first links 502, the second connecting joints 601 are hinged with the second links 602, the first connecting joint 501 adjacent to the first support arm 1 is fixedly connected with the first support arm 1, the other first connecting joint 501 is fixedly connected with the third support arm 3,the second connecting joint 601 adjacent to the second support arm 2 is fixedly connected with the second support arm 2 and is rotatably connected with the third support arm 3, wherein the other second connecting joint 601 is suitable for fixed connection with a mounting seat for mounting the surgical instrument, and in the present embodiment, the other second connecting joint 601 is suitable for fixed connection with a sliding table group 4.

Specifically, as shown in Fig. 1, the first connecting joints 501 are hinged with first links 502, so that the first parallelogram link mechanism 5 is deformable; the second connecting joints 601 are hinged with the second links 602, so that the second parallelogram link mechanism 6 is deformable; the first connecting joints 501 are fixedly connected with the end, adjacent to the second support arm 2, of the first support arm 1, meanwhile, the first connecting joints 501 are provided with bearings, the first connecting joints 501 are rotatably connected with the second support arm 2 via the bearings, that is, the first support arm 1 is rotatably connected with the second support arm 2 via the first connecting joints 501. Similarly, the second connecting joints 601 are fixedly connected with the ends, adjacent to the third support arm 3, of the second support arm 2, meanwhile, the second connecting joints 601 are provided with bearings, the second connecting joints 601 are rotatably connected with the third support arm 3 via the bearings, that is, the second support arm 2 is rotatably connected with the third support arm 3 via the first connecting joints 601. By the above-mentioned arrangement, the first support arm 1 and the third support arm 3 are synchronous, and the second support arm 2 and the sliding table group 4 are synchronous; when the second support arm 2 wings around the first support arm 1, an included angle of the second support arm 2 and the third support arm 3 varies, the first parallelogram link mechanism 5 and the second parallelogram link mechanism 6 deform, so that the sliding table group 4 and the second support arm 2 swing at the same angle, and then, the swing of the sliding table group 4 around the telecentric point 19 is achieved.

Optionally, as shown in Fig. 1, a base 7 and a multi-dimensional force sensor 8 are further included, the base 7 is suitable for connection with the multi-dimensional force sensor 8, and the base 7 is rotatably connected with the end, away from the second support arm 2, of the first support arm 1.

The multi-dimensional force sensor 8 is preferably a six-dimensional force sensor, namely a sensor capable of measuring three force components and three moment components at the same time. As shown in Fig. 1, the base 7 is rotatably connected with the end, away from the second support arm 2, of the first support arm 1, so that the first support arm 1 is capable of rotating around the base 7 in the circumferential direction of the base 7. The other end of the first support arm is rotatably connected with the second support arm 2, that is, the second support arm 2 is capable of rotating relative to the first support arm 1 in an axis direction of the first support arm 1.

Specifically, by rotatably connecting the base 7 with the end, away from the second support arm 2, of the first support arm 1, the sliding table group 4 may rotate by controlling the rotation of the first support arm 1, so that the rotation of the sliding table group 4 around the telecentric point 19 is achieved, and then, the activity range of the surgical instrument is widened. In addition, the base 7 is connected with the multi-dimensional force sensor 8, and therefore, the surgical robot can accurately monitor the operating force of the master arm and whether slip and vibration occur in an operation process via the multi-dimensional force sensor 8, and then, it is convenient to realize a precise surgical operation. Meanwhile, by virtue of data of the sensor, the motion of the master arm may be controlled by a power output direction and power of the master arm on the basis of a force application direction and a force when a person drags the master arm in place with a hand, thereby assisting in the person in doing dragging motion.

Optionally, as shown in Fig. 1, the master arm of the surgical robot further includes harmonic reduction gears 9, motors 10 and pulley sets 11 which are disposed on two ends of the first support arm 1, the pulley sets 11 include driving wheels and driven wheels which are connected in a driving way, the driving wheels are connected with the motors 10 in a driving way, the driven wheels are connected with the harmonic reduction gears 9 in a driving way, and two ends of the first support arm 1 are connected with the base 7 and the second support arm 2 respectively via the harmonic reduction gears 9.

Specifically, one end of the first support arm 1 is rotatably connected with the base 7 via one of the harmonic reduction gears 9, the other end thereof is rotatably connected with the second support arm 2 via the other harmonic reduction gear 9, the motors 10 are connected with the harmonic reduction gears 9 in a driving way via the pulley sets 11, then, a rotation angle of the first support arm 1 may be controlled by the motor 10 adjacent to the base 7, and a rotation angle of the second support arm 2 may be controlled by the motor 10 adjacent to the second support arm 2, wherein a motor control panel 18 is disposed in the first support arm 1, the motor control panel 18 is used for controlling the motor 10 adjacent to the base 7, the other motor control panel 18 is disposed in the second support arm 2, and the motor control panel 18 in the second support arm 2 is used for controlling the motor 10 adjacent to the second support arm 2. By such structural arrangement, control on the rotation angle of the first support arm 1 and a swing angle of the second support arm 2 is achieved, and then, control on a rotation angle and swing angle of the sliding table group 4 is achieved.

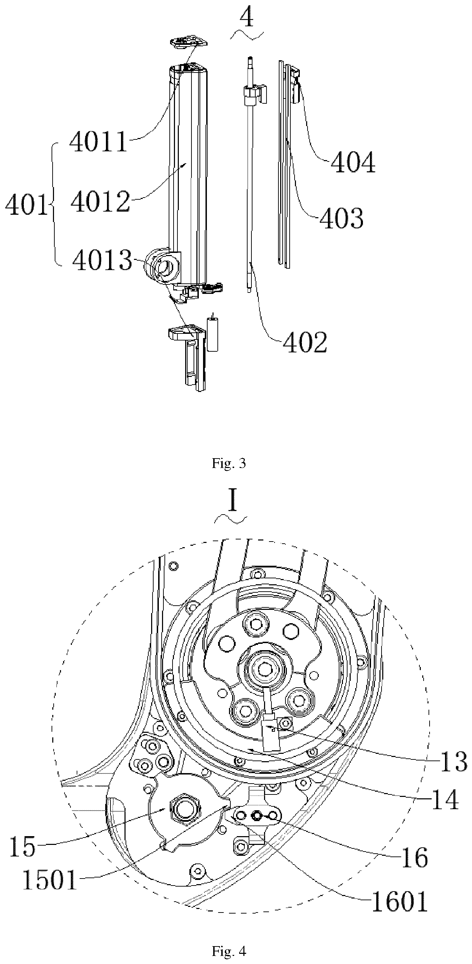

Optionally, as shown in Fig. 1, Fig. 2 and Fig. 4, the master arm of the surgical robot further includes a first photoelectric sensor 12 disposed on the first support arm 1, the base 7 is provided with a sensing coating or first sensing patch, and the first photoelectric sensor 12 and the sensing coating or the first sensing patch are matched to limit a rotation angle of the first support arm 1 relative to the base 7.

Specifically, the first support arm 1 is provided with the first photoelectric sensor 12, and then, the first photoelectric sensor 12 may rotate synchronously with the first support arm 1. When the first photoelectric sensor 12 senses the sensing coating or first sensing patch on the base 7, the motors stop driving; or when the first photoelectric sensor 12 does not sense the sensing coating or first sensing patch on the base 7, the motors stop driving, and thus, the first photoelectric sensor 12 and the sensing coating or the first sensing patch are matched to limit the rotation angle of the first support arm 1.

Optionally, as shown in Fig. 1, Fig. 2 and Fig. 4, the master arm of the surgical robot further includes a second photoelectric sensor 13 and a second sensing patch 14, the second photoelectric sensor 13 is disposed on the second support arm 2, the second sensing patch 14 is disposed on the first support arm 1, and the second photoelectric sensor 13 and the second sensing patch 14 are matched to limit a rotation angle of the second support arm 2 relative to the first support arm 1.

Specifically, the second sensing patch 14 is an arc-shaped sensing patch. When the second support arm 2 swings, the second photoelectric sensor 13 swing synchronously therewith, and then, the second photoelectric sensor 13 may swing around the second sensing patch 14. Similarly, when the second photoelectric sensor 13 senses the second sensing patch 14 on the base 7, the motors 10 stops driving; or when the second photoelectric sensor 13 does not sense the second sensing patch 14, the motors 10 stops driving. Thus, the second photoelectric sensor 13 and the second sensing patch 14 are matched to limit the rotation angle of the second support arm 2.

By limiting the rotation angle of the first support arm 1 and the swing angle of the second support arm 2, there is always an avoiding space for avoiding a patient below the third support arm 3, thereby facilitating the surgical operation.

Optionally, as shown in Fig. 1, Fig. 2 and Fig. 4, the master arm of the surgical robot further includes braking mechanisms, the braking mechanisms are disposed on a junction of the base 7 and the first support arm 1 and a junction of the first support arm 1 and the second support arm 2, the braking mechanisms include braking discs 15, braking rods 16 and first electromagnets 17, side surfaces of the braking discs 15 are provided with first protruding portions 1501, side surfaces of the braking rods 15 are provided with second protruding portions 1601, and the first electromagnets 17 are suitable for electrification to adsorb the braking rods 16 so as to enable the first protruding portions 1501 and the second protruding portions 1601 to be located on the same plane and are also suitable for enabling side surfaces of the first protruding portions 1501 and the second protruding portions 1601 to be abutted.

Specifically, the braking discs 15 are connected with the pulley sets 11, and the braking rods 16 are disposed on the base 7 and the first support arm 1 respectively. When the first electromagnets 17 are unelectrified, the first protruding portions 1501 and the second protruding portions 1601 are not located on the same plane, that is, the braking rods 16 may not disturb the rotation of the braking discs 15; and when being electrified, the first electromagnets 17 adsorb the braking rods 16, so that the first protruding portions 1501 and the second protruding portions 1601 are located on the same plane. At the moment, side surfaces of the first protruding portions 1501 and the second protruding portions 1601 are abutted or to be abutted, that is, collision occurs via the first protruding portions 1501 and the second protruding portions 1601, thereby realizing braking. Therefore, by disposing the braking mechanisms on the junction of the base 7 and the first support arm 1 and the junction of the first support arm 1 and the second support arm 2, the first support arm 1 and the second support arm 2 can be effectively braked.

Optionally, elastic members are disposed in the braking rods 16, and when the first electromagnets 17 are electrified to adsorb the braking rods 16, the elastic members elastically deform so as to drive the braking rods 16 to reset when the first electromagnets 17 are unelectrified; or
the master arm of the surgical robot further includes second electromagnets, when the first electromagnets 17 are unelectrified, the second electromagnets are suitable for electrification to adsorb the braking rods 16 to reset.

Specifically, when the first electromagnets 17 are unelectrified, the braking rods 16 need to reset, thereby preventing the first protruding portions and the second protruding portions from being further abutted for braking. Therefore, the elastic members such as springs may be disposed in the braking rods 16, and when the first electromagnets 17 are unelectrified, the braking rods 16 reset under the action of elastic forces of the elastic members. Or, the second electromagnets are disposed, and the second electromagnets are electrified to adsorb the braking rods 16 so that the braking rods 16 reset, wherein when the first electromagnets 17 are electrified, the second electromagnets are unelectrified; and when the first electromagnets 17 are unelectrified, the second electromagnets 17 are electrified.

Optionally, as shown in Fig. 2, Fig. 3 and Fig. 5, a sliding table group 4 is further included, the other end of the third support arm 3 is rotatably connected with the sliding table group 4, the other end of the second parallelogram link mechanism 6 is connected with the sliding table group 4, the sliding table group 4 includes sliding tables 401, lead screws 402, guide rails 403 and mounting tables 404, the sliding tables 401 are rotatably connected with the third support arm 3 and are fixedly connected with the second parallelogram link mechanism 6, two ends of the lead screws 402 are rotatably connected with the sliding tables 401, the guide rails 403 are fixed to the sliding tables 401, nuts of the lead screws 402 are fixedly connected with the mounting tables 404, the mounting tables 404 are slidably connected with the guide rails 403, and the mounting tables 404 are suitable for mounting surgical instruments.

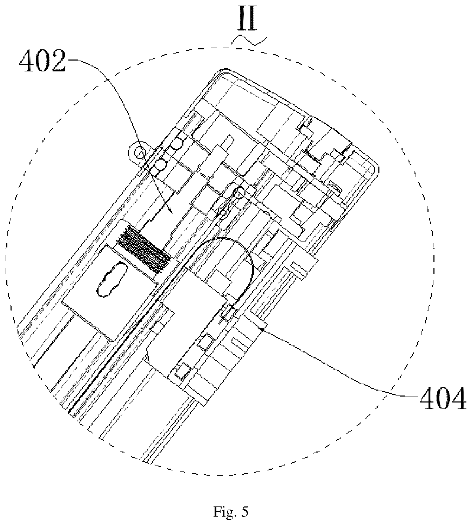

The other end of the third support arm 3 is rotatably connected with the sliding table group 4, and the other end of the second parallelogram link mechanism 6 is connected with the sliding table group 4, so that the second support arm 2 and the sliding table group 4 swing synchronously under the limit of the second parallelogram link mechanism 6, and then, the synchronous swing of the surgical instrument and the second support arm 2 is achieved. Specifically, as shown in Fig. 5, the sliding tables 401 include sliding table bodies 4012, upper sliding table covers 4011 and lower sliding table covers 4013, the upper sliding table covers 4011 are detachably connected to the upper ends of the sliding table bodies 4012, the lower sliding table covers 4013 are detachably connected to the lower ends of the sliding table bodies 4012, cavities are disposed in the sliding table bodies 4012, and the cavities are used for accommodating the lead screws 402 and the guide rails 403, wherein two ends of the lead screws 402 are rotatably connected with the sliding tables 401. The motors 10 and the pulley sets 11 are further disposed in the sliding tables 401, the motors 10 drive the lead screws 402 to rotate via the pulley sets 11. The lead screws 402 are of mechanical structures converting rotational motion into linear motion, the nuts of the lead screws 402 are fixedly connected with the mounting tables 404, the mounting tables 404 are slidably connected with the sliding tables 401, and therefore, when the motors 10 drive shafts of the lead screws 402 to rotate, the nuts on the lead screws 402 may not rotate therewith under the limit of the mounting tables 404, but do linear motion relative to the shafts of the lead screws, thereby driving the mounting tables 404 to do linear motion on the guide rails 403, and then, achieving linear motion of the surgical instrument.

Another embodiment of the present invention provides a surgical robot including the above-mentioned master arm of the surgical robot.

Compared with the prior art, the surgical robot in the present embodiment has the same beneficial effects as the master arm of the surgical robot, the descriptions thereof are omitted herein.

Although the present disclosure is described as above, the protection scope of the present disclosure is not limited thereto. Various alterations and modifications may be made by the skilled in the art without departing from the spirit and scope of the present disclosure, and these alternations and modifications fall within the protection scope of the present invention.

## Claims

1. A master arm of a surgical robot, comprising a first support arm (1), a second support arm (2), a third support arm (3), a first parallelogram link mechanism (5) and a second parallelogram link mechanism (6), one end of the first support arm (1) being rotatably connected with one end of the second support arm (2), the other end of the second support arm (2) being rotatably connected with one end of the third support arm (3), two ends of the first parallelogram link mechanism (5) being connected with the first support arm (1) and the third support arm (3) respectively, and one end of the second parallelogram link mechanism (6) being connected with the second support arm (2);
wherein the first parallelogram link mechanism (5) and the second parallelogram link mechanism (6) are suitable for deforming with the swing of the second support arm (2).

2. The master arm of the surgical robot of claim 1, wherein the first parallelogram link mechanism (5) comprises two parallel first connecting joints (501) and two parallel first links (502), the second parallelogram link mechanism (6) comprises two parallel second connecting joints (601) and two parallel second links (602), the first connecting joints (501) are hinged with the first links (502), the second connecting joints (601) are hinged with the second links (602), the first connecting joint (501) adjacent to the first support arm (1) is fixedly connected with the first support arm (1), the other first connecting joint (501) is fixedly connected with the third support arm (3), and the second connecting joint (601) adjacent to the second support arm (2) is fixedly connected with the second support arm (2) and is rotatably connected with the third support arm (3).

3. The master arm of the surgical robot of claim 1 or 2, further comprising a base (7) and a multi-dimensional force sensor (8), the base (7) being suitable for connection with the multi-dimensional force sensor (8), and the base (7) being rotatably connected with the end, away from the second support arm (2), of the first support arm (1).

4. The master arm of the surgical robot of claim 3, further comprising harmonic reduction gears (9), motors (10) and pulley sets (11) which are disposed on two ends of the first support arm (1), the pulley sets (11) comprising driving wheels and driven wheels which are connected in a driving way, the driving wheels being connected with the motors (10) in a driving way, the driven wheels being connected with the harmonic reduction gears (9) in a driving way, and two ends of the first support arm (1) being connected with the base (7) and the second support arm (2) respectively via the harmonic reduction gears (9).

5. The master arm of the surgical robot of claim 3, further comprising a first photoelectric sensor (12) disposed on the first support arm (1), the base (7) being provided with a sensing coating or first sensing patch, and the first photoelectric sensor (12) and the sensing coating or the first sensing patch being matched to limit a rotation angle of the first support arm (1) relative to the base (7).

6. The master arm of the surgical robot of claim 1 or 2, further comprising a second photoelectric sensor (13) and a second sensing patch (14), the second photoelectric sensor being disposed on the second support arm (2), the second sensing patch (14) being disposed on the first support arm (1), and the second photoelectric sensor (13) and the second sensing patch (14) being matched to limit a rotation angle of the second support arm (2) relative to the first support arm (1).

7. The master arm of the surgical robot of claim 3, further comprising braking mechanisms, the braking mechanisms being disposed on a junction of the base (7) and the first support arm (1) and a junction of the first support arm (1) and the second support arm (2), the braking mechanisms comprising braking discs (15), braking rods (16) and first electromagnets (17), side surfaces of the braking discs (15) being provided with first protruding portions (1501), side surface of the braking rods (16) being provided with second protruding portions (1601), and the first electromagnets (17) being suitable for electrification to adsorb the braking rods (16) so as to enable the first protruding portions (1501) and the second protruding portions (1601) to be located on the same plane and being also suitable for enabling side surfaces of the first protruding portions (1501) and the second protruding portions (1601) to be abutted.

8. The master arm of the surgical robot of claim 7, wherein elastic members are disposed in the braking rods (16), and when the first electromagnets (17) are electrified to adsorb the braking rods (16), the elastic members elastically deform so as to drive the braking rods (16) to reset when the first electromagnets (17) are unelectrified; or
second electromagnets are further comprised, when the first electromagnets (17) are unelectrified, the second electromagnets are suitable for electrification to adsorb the braking rods (16) so that the braking rods (16) reset.

9. The master arm of the surgical robot of claim 1 or 2, further comprising a sliding table group (4), the other end of the third support arm (3) being rotatably connected with the sliding table group (4), the other end of the second parallelogram link mechanism (6) being connected with the sliding table group (4), the sliding table group (4) comprising sliding tables (401), lead screws (402), guide rails (403) and mounting tables (404), the sliding tables (401) being rotatably connected with the third support arm (3) and being fixedly connected with the second parallelogram link mechanism (6), two ends of the lead screws (402) being rotatably connected with the sliding tables (401), the guide rails (403) being fixed to the sliding tables (401), nuts of the lead screws (402) being fixedly connected with the mounting tables (404), the mounting tables (404) being slidably connected with the guide rails (403), and the mounting tables (404) being suitable for mounting surgical instruments.

10. A surgical robot, comprising the master arm of the surgical robot of any one of claims 1 to 9.
